# EUROPEAN PATENT SPECIFICATION

(11) **EP 1 311 211 B1**
(45) Date of publication and mention of the grant of the patent: **23.02.2005**
(21) Application number: 01976092.5
(22) Date of filing: 16.08.2001
(51) Int. Cl.: A61F 2/16

(54) **ROLLABLE INTRAOCULAR LENS**
EINROLLBARE INTRAOKULARLINSE
LENTILLE INTRAOCULAIRE POUVANT SE ROULER

(30) Priority: 17.08.2000 US 225955 P
(43) Date of publication of application: 21.05.2003
(73) Proprietor: IOLTECH, 17180 Perigny (FR); Novartis Pharma GmbH, 1230 Wien (AT)
(72) Inventor: MORGAN, Courtney, Flem, Alpharetta, GA 30222 (US); MORGAN, Drew, Anderson, Woodstock, GA 30188 (US)
(74) Representative: Lewitter, Herbert
(86) International application number: PCT/EP2001/009464
(87) International publication number: WO 2002/013728

(56) References cited:
- WO-A-94/18908
- US-A- 4 834 750
- US-A- 5 331 073

## Description

### Field of the Invention

The present invention relates generally to optical lenses, and more particularly to a rollable intraocular lens having an improved haptic attachment providing reduced edge and overall lens thickness, thereby enabling the lens to be rolled more compactly for implantation.

### Description of Related Art

Intraocular lenses (IOL's) are artificial optical lenses that are surgically implanted to replace a diseased or damaged natural lens of an eye. IOL's may be implanted in the anterior chamber or the posterior chamber of the eye, and commonly incorporate a lens body and one or more haptics for positioning and support of the implanted lens. Haptics can take the form of filaments attached to a separate lens body, or alternatively can comprise integral extensions of the lens body. It is desirable that the haptic be securely affixed to the lens body in order to reduce the likelihood of detachment during the implantation procedure or in use.

Numerous intraocular lenses have been described in prior art. For example, WO 94/18908 discloses a rollable intraocular lens having a periphery with a reduced thickness.

In order to minimize the size of the incision necessary to surgically implant an IOL, and thereby reduce associated trauma and recovery time, it has been found advantageous to form the IOL of a flexible material that permits folding or rolling of the lens to reduce its insertion profile. As used herein, the "insertion profile" of a lens refers to the minimum opening size through which a rolled IOL can be passed for implantation. Generally, and with all other factors being equal, the more compactly an IOL can be rolled or folded, the smaller its resulting insertion profile and, accordingly, the smaller the incision that is required for implantation.

It has been discovered, however, that regardless of how tightly an IOL is rolled, the amount of material forming the lens (i.e., the volume of space occupied by the lens) limits the minimum attainable insertion profile. Additionally, because a thinner lens is typically more flexible than a thicker lens of similar material, a thinner lens can generally be rolled more tightly than a thicker lens. Thus, it would be desirable to minimize the thickness and overall amount of material used to form a rollable IOL. The need for a secure attachment between the haptic and the lens body of previously known rollable IOLs, however, typically requires that the outer periphery of an JOL have a thickness at least sufficient to engage the haptic that the outer periphery of an IOL have a thickness at least sufficient to engage the haptic with the edge of the lens body. For example, a haptic filament commonly is secured within a haptic bore formed in the edge of the lens body. As a result, the peripheral edge of previously known rollable IOLs of this variety typically has a minimum thickness of no less than the thickness of the haptic filament plus a thickness of lens material surrounding the haptic bore sufficient to secure the haptic filament therein.

In order to provide the desired optical performance, the central optic zone of an IOL typically comprises a curved surface on one or both of its faces, generating a convex body having a central thickness substantially greater than the thickness of the peripheral edge. Accordingly, the peripheral edge thickness necessary to secure a haptic to the lens body has heretofore been considered a limiting factor in previous efforts to minimize the insertion profile of a rollable IOL. Thus, it has been discovered that a need exists for a rollable IOL having a reduced peripheral edge thickness, thereby enabling a reduction in overall lens thickness, yet providing secure attachment between a haptic and the lens body. It is also desirable to provide a rollable IOL enabling a reduction in the overall lens thickness that is required to provide specified optical characteristics.

It is to the provision of a rollable IOL meeting these and other needs that the present invention is primarily directed.

### Summary of the Invention

The present invention provides an improved intraocular lens, and related methods of fabrication. The improved intraocular lens of the present invention is rollable, thereby reducing the insertion profile of the lens and associated trauma and recovery time. The lens of the present invention includes one or more flared portions along its peripheral edge for providing secure attachment between a haptic and the lens body. Between these flared portions, the peripheral edge of the intraocular lens of the present invention is relatively thin, thereby reducing the mass of the lens and the amount of material used to form the lens, and further reducing the insertion profile of the lens. Also, because the peripheral edge thickness of the lens is reduced as compared to previously known lenses, sufficient curvature can be provided to the lens surface(s) to produce a desired optical characteristic in a thinner, more flexible lens than was previously possible. The reduction in mass and thickness of the peripheral edge and the resulting increased flexibility advantageously further reduce the insertion profile of the lens, and enable the provision of improved optical performance by a lens of a given overall thickness.

In one aspect, the invention relates to a rollable intraocular lens comprising a flexible lens body having first and second faces intersecting at a peripheral edge, at least one of said first and second faces being generally convex, whereby said lens body has a central thickness and an edge thickness, the central thickness being greater than the peripheral edge thickness; said lens body further comprising at least one flared portion along said peripheral edge, said flared portion having a thickness greater than the remaining peripheral edge thickness.

In further preferred embodiments, the rollable intraocular lens includes at least one haptic element attached to the flexible lens body at the flared portion. The haptic element is preferably engaged within a haptic bore formed in the flared portion of the flexible lens body, and the haptic element is preferably thermally welded within the haptic bore. In another further preferred embodiment, the flexible lens body comprises a shape memory material, such as a modified poly(methyl methacrylate) (PMMA).

In another aspect, the invention relates to a an intraocular lens comprising:
a lens body having first and second faces, a peripheral edge, and first and second flared portions spaced from one another along said peripheral edge;
a first haptic element attached to said flexible lens body at said first flared portion; and
a second haptic element attached to said flexible lens body at said second flared portion; wherein said lens body is foldable along an axis between said first and second flared portions.

In another aspect, the invention is a method of forming an intraocular lens. The method preferably includes: (1) providing a flexible lens body having a predetermined maximum overall thickness; (2) forming a central optic zone in the flexible lens body, the central optic zone having at least one convex face having a predetermined degree of curvature to define a central thickness between the at least one convex face and an opposed second face; and (3) forming a peripheral zone in the flexible lens body surrounding the central optic zone, the peripheral zone having an edge thickness less than the central thickness, and the peripheral zone further having at least one flared portion with a thickness greater than the remaining peripheral edge thickness.

These and other features and advantages of the present invention are described herein with reference to example embodiments shown in the appended drawing figures.

### Brief description of the drawing figures

Figure 1 is a top view of an intraocular lens according to a preferred form of the present invention.
Figure 2 is a perspective view of a portion of the intraocular lens of Fig. 1, shown in greater detail.
Figure 3 is a cross-sectional side view of the intraocular lens of Fig. 1, taken along section line 3-3.
Figure 4 is a cross-sectional side view of the intraocular lens of Fig. 1, taken along section line 4-4.
Figure 5 is a detailed side view of a portion of an intraocular lens according to one form of the present invention.
Figure 6 is a detailed side view of a portion of an intraocular lens according to another form of the present invention.
Figure 7 is a perspective view of a folded or rolled intraocular lens according to a preferred form of the present invention.
Figure 8 is a top view of an intraocular lens according to another preferred form of the present invention.

### Detailed Description

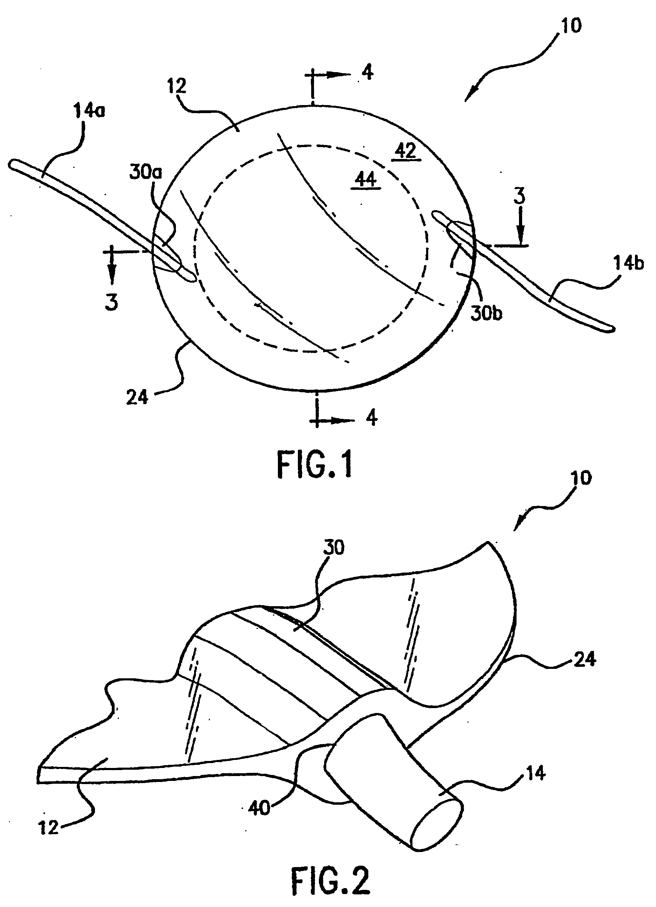

Referring now to the drawing figures, wherein like reference numerals represent like parts throughout, preferred forms of the present invention will now be described. Figures 1-4 show a rollable intraocular lens (IOL) 10 according to a preferred form of the present invention. The intraocular lens 10 preferably comprises a lens body 12 with at least one haptic element 14 attached thereto. In the depicted embodiment, two haptic elements 14 are provided, with a first haptic element 14a and a second haptic element 14b arranged generally diametrically opposite one another. In alternate embodiments, a single haptic element 14 can be attached to the lens body 12 or three, four or more haptic elements can be spaced about the circumference of the lens body 12.

In preferred form, the lens body 12 is formed from a flexible, transparent polymeric material such as, for example, modified poly(methyl methacrylate) (PMMA), modified PMMA hydrogels, silicone, or other polymeric materials. In a particularly preferred embodiment, the lens body 12 is formed of a shape-memory material such as is disclosed by U.S. Patent No. 4,731,079. The lens body can be frozen in a rolled configuration and, upon exposure to body temperature during implantation, will return under its own influence to an unrolled configuration. The haptics 14 are preferably flexible filaments formed of polypropylene or other materials.

As seen best with reference to Figs. 1, 3 and 4, the lens body 12 is preferably a generally cylindrical, disc-shaped component comprising a first face 20 and a second face 22, the first and second faces intersecting at a peripheral edge 24. At least one of the first and second faces 20, 22 is preferably generally convex, having the degree of curvature required to provide the lens with a specified optical refraction. In one embodiment of the invention, the first and second faces 20, 22 are both convex, thereby forming a bi-convex or convex-convex lens. Alternatively, one face is convex and the other is planar, thereby forming a piano-convex lens. In yet another alternate form, one face is convex and the other is concave, thereby forming a convex-concave lens. The lens body has a central thickness *t*_{*c*}, measured between the first and second faces 20, 22 proximal the center of the lens body 12, and an edge thickness *t*_{*e*}, measured between the first and second faces proximal the peripheral edge 24. Preferably, the central thickness *t*_{*c*} is greater than the edge thickness *t*_{*e*}.

The lens body 12 further comprises at least one flared portion 30 along the peripheral edge 24, as seen best with reference to Figs. 2-6. In a preferred embodiment, first and second flared portions 30a, 30b are provided along the peripheral edge 24, generally diametrically opposite one another. In alternate embodiments, a single flared portion, or portion, or three or more flared portions are provided. Each flared portion has a thickness *t*_{*f*}, which is greater than the edge thickness *t*_{*e*}. The thickness *t*_{*f*} of the flared portion 30 is preferably less than the central thickness *t*_{*c*}, whereby the maximum overall thickness of the lens body 12 is equal to the central thickness *t*_{*c*}. Altematively, the thickness *t*_{*f*} of the flared portion 30 is approximately equal to or greater than the central thickness *t*_{*c*}.

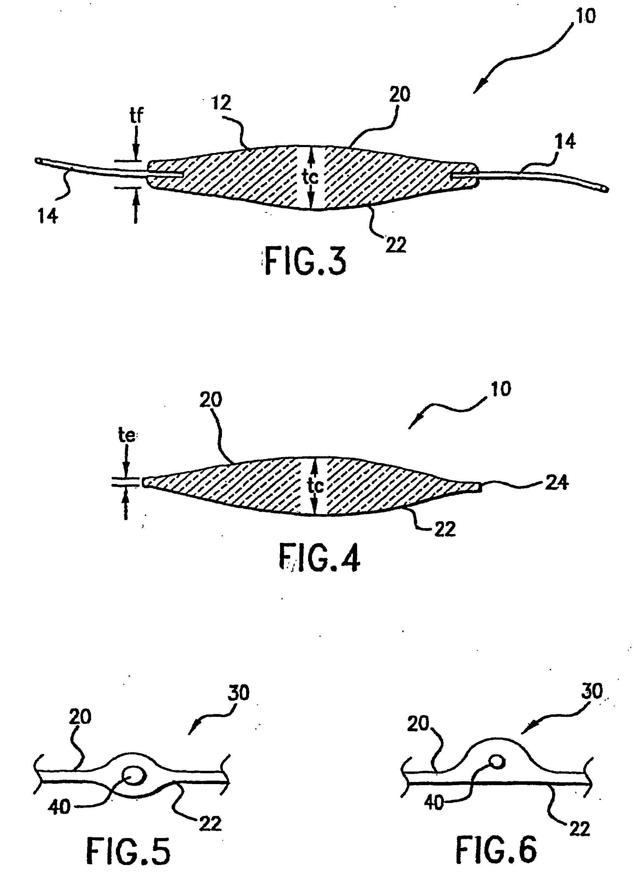

In one embodiment, depicted in detail by Fig. 5, the flared portion 30 is expanded relative to the edge thickness *t*_{*e*} outwardly from both the first and second faces 20, 22. In another embodiment, depicted in detail by Fig. 6, the flared portion 30 is expanded relative to the edge thickness *t*_{*e*} outwardly from only the first face 20, and is generally co-planar with the second face 22. A smooth transition, such as a fillet, is preferably provided between the peripheral edge 24 and each flared portion 30, to minimize any stress concentrations during flexure of the lens and to provide a smoother surface during implantation.

Each flared portion 30 preferably defines a haptic bore 40 formed therein for receiving and retaining a haptic element 14. As seen best with reference to Figs. 1-3 each haptic bore 40 extends a depth into the lens body 12, generally radially or chordally from the circumferential edge of the lens body at the flared portion 30. Preferably, the depth of the haptic bore 40 is limited to the peripheral zone 42 (outside the broken indicator line of Fig. 1) of the lens body 12, and does not extend into the central optic zone 44 (inside the broken indicator line of Fig. 1) to a point where it would be visually perceptible in use. A haptic 14, such as a polypropylene filament, is received in the haptic bore 40 and secured therein, as by welding, adhesives, compression fit, or other attachment means. In an example embodiment, laser energy is used to thermally weld a haptic 14 in place within the haptic bore 40. Each flared portion 30 preferably has a thickness *t*_{*f*} somewhat greater than the diameter of the haptic 14, whereby a sufficient thickness of lens body material surrounds the haptic bore 40 to provide secure attachment of the haptic 14 to the lens body 12. For example, the thickness *t*_{*f*} is preferably at least about one and one-half to two or more times the diameter of the haptic 14.

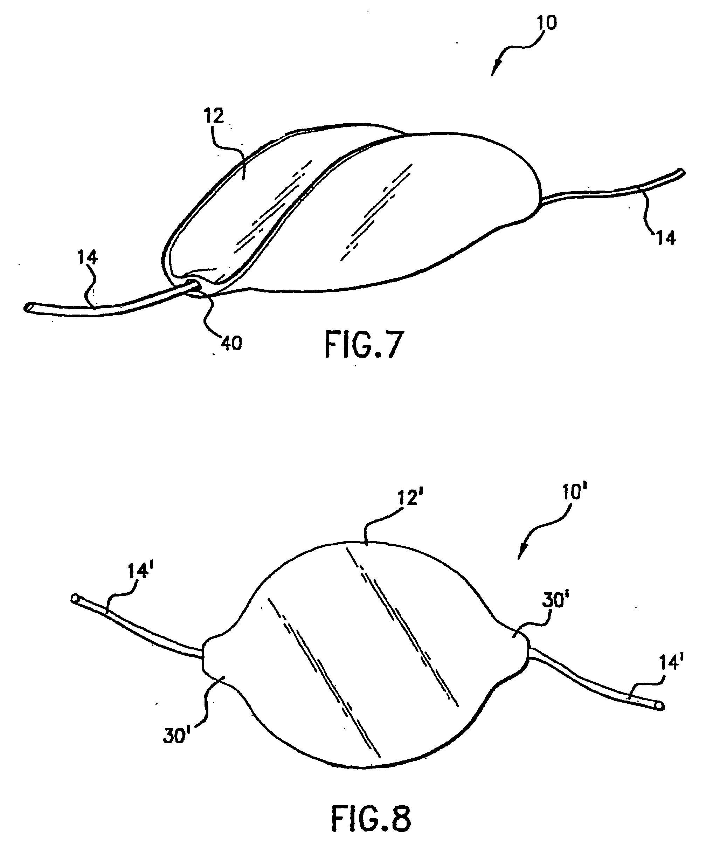

Figure 8 shows an alternate embodiment of the intraocular lens 10', according to another form of the invention. In this embodiment, the peripheral zone of the lens body 12' is substantially eliminated between the flared portions 30', thereby further reducing the mass and amount of material of the intraocular lens 10'. One or more haptics 14' are attached to the lens body 12' at flared portions 30' in substantially the manner described above.

Forming the lens body 12 of a flexible material allows the intraocular lens 10 of the present invention to be rolled (folded), for example, as shown in Fig. 7. Rolling the lens reduces its insertion profile, permitting implantation of the lens through a smaller incision in the eye than would be possible with a non-rollable lens. For example, if the lens body 12 were not flexible, insertion of the lens 10 would typically require an incision of at least the diameter of the lens body, whereas by rolling the lens body, the lens can be inserted through a considerably smaller incision of, for example, approximately one-half the diameter of the lens body, or smaller. Because the thickness of the peripheral zone 42 is minimized between the first and second flared portions 30a, 30b, the overall mass of the lens body 12 according to the present invention is reduced relative to previously known rollable lenses. Also, the overall thickness of the lens body 12 that is necessary to provide the surface curvature required to produce a specified optical characteristic is reduced relative to previously known rollable lenses. As a result, the flexibility of the lens body 12 is increased, and the lens of the present invention can be rolled more tightly and compactly than previously known rollable lenses. The lens body 12 is preferably rolled or folded along a haptic-to-haptic axis extending between first and second flared portions 30a, 30b. By folding the lens body 12 in this manner, the thinner (and most flexible) edge portions of the lens body are subject to the greatest degree of flexure, thereby further minimizing the insertion profile of the lens, whereas the thicker (and least flexible) portions of the lens lying along the haptic-to-haptic axis are subject to the least degree of flexure.

The present invention further comprises a method of forming an intraocular lens 10. A flexible lens body is preferably provided, for example, by molding the lens body of a flexible material. Most preferably, the flexible material is a shape memory material such as modified poly(methyl methacrylate). A central optic zone is formed in the flexible lens body, the central optic zone preferably comprising at least one convex face having a predetermined degree of curvature to define a central thickness between the at least one convex face and an opposed second face. A peripheral zone is also formed in the flexible lens body surrounding the central optic zone, the peripheral zone comprising an edge thickness less than the central thickness. The peripheral zone preferably further comprises at least one flared portion having a thickness greater than the edge thickness. In a further embodiment, the fabrication method of the present invention further comprises attaching a haptic element to the at least one flared portion of the lens body. In a still further preferred embodiment, the flexible lens body comprises a shape memory material, and the intraocular lens is rolled into a compact configuration and temporarily fixed in the compact configuration for implantation.

The method of forming a lens according to the present invention preferably comprises a molding operation and/or a lathe cutting operation. For example, at least one of the steps of: (1) forming a central optic zone in the flexible lens body; and (2) forming a peripheral zone in the flexible lens body can comprise turning and cutting the lens body on a lathe, and polishing the lens as needed. Alternatively or additionally, at least one of the steps of: (1) providing a flexible lens body having a predetermined maximum overall thickness; (2) forming a central optic zone in the flexible lens body; and (3) forming a peripheral zone in the flexible lens body, can comprise molding a shaped lens body, as for example, in a double-sided mold, and curing the lens. In an example embodiment, the lens body is formed using a non-axisymmetric lathe cutting operation. For example, a lathe having a rapidly positionable, oscillating cutting tool, can be utilized to shape the lens body from a lens blank. A flexible lens blank is preferably formed of a specified lens material, such as modified shape-memory PMMA, as by molding. The lens blank is mounted to the spindle of the lathe and turned. The blank is cut using the oscillating cutting tool to form a lens body having at least one optical face and at least one flared portion along the peripheral edge of the lens body. The haptic bores are preferably formed into the lens body by molding or drilling, and the haptics are inserted and attached therein by welding, adhesive or other attachment means. In alternate embodiments, the intraocular lens of the present invention is formed by milling, cutting, or by other machining or fabrication methods.

Because the present invention eliminates the need for a minimum edge thickness that is sufficient to secure a haptic therein, the lens designer can adjust the edge thickness of a lens to suit the desired optical characteristic of the lens, thereby providing an additional degree of freedom in designing a lens. For example, a lens body having a predetermined or standard maximum overall thickness can be provided, and the required degree of curvature for a specified optical characteristic can be generated without regard for the resulting minimum edge thickness. Likewise, a minimum edge thickness (which can be less than, equal to, or greater than the haptic diameter) can be specified, and the maximum overall thickness of the lens determined by the required degree of curvature for a specified optical characteristic.

While the invention has been described in its preferred forms, it will be readily apparent to those of ordinary skill in the art that many additions, modifications and deletions can be made thereto without departing from the scope of the invention.

## Claims

1. A rollable intraocular lens comprising a flexible lens body having first and second faces intersecting at a peripheral edge, at least one of said first and second faces being generally convex, said lens body having a central thickness and a peripheral edge thickness, the central thickness being greater than the peripheral edge thickness, **characterized in that** said lens body comprises at least one flared portion (30) along said peripheral edge, said flared portion having a thickness greater than the remaining peripheral edge thickness.

2. The rollable intraocular lens of Claim 1, **characterized in that** at least one haptic element is attached to said flexible lens body at said flared portion.

3. The rollable intraocular lens of Claim 2, **characterized in that** said haptic element is thermally welded within a haptic bore formed in said flared portion of said flexible lens body.

4. The rollable intraocular lens of any one of the prededing claims, **characterized in that** said flexible lens body comprises a shape memory material.

5. The rollable intraocular lens of any one of the preceding claims, **characterized in that** said flexible lens body is formed from modified poly(methyl methacrylate).

6. The rollable intraocular lens of any one of the preceding claims, **characterized in that** said lens comprises a smooth transition between said peripheral edge and each said flared portion.

7. The rollable intraocular lens of any one of the preceding claims, **characterized in that** said flared portion has a thickness less than the central thickness.

8. The rollable intraocular lens of any one of the preceding claims, **characterized in that** said lens body has first and second flared portions spaced from one another along said peripheral edge;
a first haptic element attached to said flexible lens body at said first flared portion; and
a second haptic element attached to said flexible lens body at said second flared portion; wherein said lens body is foldable along an axis between said first and second flared portions.

9. The intraocular lens of Claim 7 or 8, **characterized in that** said lens body comprises a smooth transition between said peripheral egde and each flared portion.

10. A method of forming an intraocular lens, said method comprising:
(1) providing a flexible lens body having a predetermined maximum overall thickness;
(2) forming a central optic zone in the flexible lens body, the central optic zone comprising at least one convex face having a predetermined degree of curvature to define a central thickness between the at least one convex face and an opposed second face; and
(3) forming a peripheral zone in the flexible lens body surrounding the central optic zone, the peripheral zone comprising an edge thickness less than the central thickness, **characterized in that** the peripheral zone comprises at least one flared portion having a thickness greater than the remaining peripheral edge thickness.

11. The method of Claim 10, **characterized in that** a haptic element is attached to the at least one flared portion of the lens body.

12. The method of Claim 10 or 11, **characterized in that** at least one of the steps of forming a central optic zone in the flexible lens body and forming a peripheral zone in the flexible lens body comprise turning and cutting the lens body on a lathe.

13. The method of Claim 10 or 11, **characterized in that** at least one of the steps of providing a flexible lens body having a predetermined maximum overall thickness, forming a central optic zone in the flexible lens body, and forming a peripheral zone in the flexible lens body comprise molding a shaped lens body.

14. The method of any one of claims 10 to 13, **characterized in that** the flexible lens body comprises a shape memory material, and wherein said method further comprises rolling the intraocular lens into a compact configuration and temporarily fixing the lens in the compact configuration for implantation.

## Patentansprüche

1. Rollbare Intraokularlinse, die einen biegsamen Linsenkörper mit einer ersten und einer zweiten Fläche, die sich an einer Umfangskante schneiden, umfasst, wobei die erste und/oder die zweite Fläche im Allgemeinen konvex sind, wobei der Linsenkörper eine mittige Dicke und eine Umfangskantendicke besitzt, wobei die mittige Dicke größer als die Umfangskantendicke ist, **dadurch gekennzeichnet, dass** der Linsenkörper längs der Umfangskante wenigstens einen erweiterten Abschnitt (30) aufweist, der eine Dicke besitzt, die größer als die Dicke der übrigen Umfangskante ist.

2. Rollbare Intraokularlinse nach Anspruch 1, **dadurch gekennzeichnet, dass** an dem biegsamen Linsenkörper am erweiterten Abschnitt wenigstens ein haptisches Element befestigt ist.

3. Rollbare Intraokularlinse nach Anspruch 2, **dadurch gekennzeichnet, dass** das haptische Element innerhalb einer haptischen Bohrung, die in dem erweiterten Abschnitt des biegsamen Linsenkörpers ausgebildet ist, thermisch verschweißt ist.

4. Rollbare Intraokularlinse nach irgendein der vorhergehenden Ansprüche, **dadurch gekennzeichnet, dass** der biegsame Linsenkörper einen Werkstoff mit Formerinnerungsvermögen umfasst.

5. Rollbare Intraokularlinse nach irgendein der vorhergehenden Ansprüche, **dadurch gekennzeichnet, dass** der biegsame Linsenkörper aus modifiziertem Poly(Methylmethacrylat) gebildet ist.

6. Rollbare Intraokularlinse nach irgendein der vorhergehenden Ansprüche, **dadurch gekennzeichnet, dass** die Linse zwischen der Umfangskante und jedem erweiterten Abschnitt einen gleichmäßigen Übergang aufweist.

7. Rollbare Intraokularlinse nach irgendein der vorhergehenden Ansprüche, **dadurch gekennzeichnet, dass** der erweiterte Abschnitt eine Dicke besitzt, die geringer als die mittige Dicke ist.

8. Rollbare Intraokularlinse nach irgendein der vorhergehenden Ansprüche, **dadurch gekennzeichnet, dass** der Linsenkörper einen ersten und einen zweiten erweiterten Abschnitt besitzt, die längs der Umfangskante voneinander beabstandet sind;
an dem biegsamen Linsenkörper am ersten erweiterten Abschnitt ein erstes haptisches Element befestigt ist; und
an dem biegsamen Linsenkörper an dem zweiten erweiterten Abschnitt ein zweites haptisches Element befestigt ist;
wobei der Linsenkörper längs einer Achse zwischen dem ersten und dem zweiten erweiterten Abschnitt faltbar ist.

9. Intraokularlinse nach einem der Ansprüche 7 oder 8, **dadurch gekennzeichnet, dass** der Linsenkörper zwischen der Umfangskante und jedem erweiterten Abschnitt einen gleichmäßigen Übergang aufweist.

10. Verfahren zum Bilden einer Intraokularlinse, wobei das Verfahren umfasst:
(1) Vorsehen eines biegsamen Linsenkörpers, der eine vorgegebene maximale Gesamtdicke hat;
(2) Bilden einer mittigen optischen Zone in dem biegsamen Linsenkörper, wobei die mittige optische Zone zumindest eine konvexe Fläche aufweist, die einen vorgegebenen Krümmungsradius besitzt, um zwischen der wenigstens einen konvexen Fläche und einer gegenüberliegenden zweiten Fläche eine mittige Dicke zu definieren; und
(3) Bilden einer Umfangszone in dem biegsamen Linsenkörper, die die mittige optische Zone umgibt, wobei die Umfangszone eine Kantendicke besitzt, die geringer als die mittige Dicke ist, **dadurch gekennzeichnet, dass** die Umfangszone wenigstens einen erweiterten Abschnitt aufweist, dessen Dicke größer als jene der verbleibenden Umfangskante ist.

11. Verfahren nach Anspruch 10, **dadurch gekennzeichnet, dass** an dem wenigstens einen erweiterten Abschnitt des Linsenkörpers ein haptisches Element befestigt ist.

12. Verfahren nach Anspruch 10 oder 11, **dadurch gekennzeichnet, dass** wenigstens einer der Schritte des Bildens einer mittigen optischen Zone in dem biegsamen Linsenkörper und des Bildens einer Umfangszone in dem biegsamen Linsenkörper das Drehen und Schneiden des Linsenkörpers auf einer Drehbank umfasst.

13. Verfahren nach Anspruch 10 oder 11, **dadurch gekennzeichnet, dass** wenigstens einer der Schritte des Vorsehens eines biegsamen Linsenkörpers mit einer vorgegebenen maximalen Gesamtdicke, des Bildens einer mittigen optischen Zone in dem biegsamen Linsenkörper und des Biegens einer Umfangszone in dem biegsamen Linsenkörper das Gießen eines geformten Linsenkörpers umfasst.

14. Verfahren nach einem der Ansprüche 10 bis 13, **dadurch gekennzeichnet, dass** der biegsame Linsenkörper einen Werkstoff mit Formerinnerungsvermögen aufweist, wobei das Verfahren ferner das Rollen der Intraokularlinse in eine kompakte Konfiguration und das vorübergehende Fixieren der Linse in der kompakten Konfiguration, um sie zu implantieren, umfasst.

## Revendications

1. Lentille intraoculaire pouvant se rouler comportant un corps de lentille souple ayant des première et seconde faces se croisant à un bord périphérique, au moins l'une desdites première et seconde faces étant globalement convexes, ledit corps de lentille ayant une épaisseur au centre et une épaisseur au bord périphérique, l'épaisseur au centre étant plus grande que l'épaisseur au bord périphérique, **caractérisée en ce que** ledit corps de lentille comporte au moins une partie évasée (30) le long dudit bord périphérique, ladite partie évasée ayant une épaisseur plus grande que l'épaisseur au bord périphérique restant.

2. Lentille intraoculaire pouvant se rouler selon la revendication 1, **caractérisée en ce qu'**au moins un élément haptique est attaché audit corps de lentille souple au niveau de ladite partie évasée.

3. Lentille intraoculaire pouvant se rouler selon la revendication 2, **caractérisée en ce que** ledit élément haptique est soudé thermiquement à l'intérieur d'un alésage haptique formé dans ladite partie évasée dudit corps de lentille souple.

4. Lentille intraoculaire pouvant se rouler selon l'une quelconque des revendications précédentes, **caractérisée en ce que** ledit corps de lentille souple comprend une matière à mémoire de forme.

5. Lentille intraoculaire pouvant se rouler selon l'une quelconque des revendications précédentes, **caractérisée en ce que** ledit corps de lentille souple est formé de poly(méthacrylate de méthyle) modifié.

6. Lentille intraoculaire pouvant se rouler selon l'une quelconque des revendications précédentes, **caractérisée en ce que** ladite lentille comporte une transition douce entre ledit bord périphérique et chaque partie évasée.

7. Lentille intraoculaire pouvant se rouler selon l'une quelconque des revendications précédentes, **caractérisée en ce que** ladite partie évasée a une épaisseur inférieure à l'épaisseur au centre.

8. Lentille intraoculaire pouvant se rouler selon l'une quelconque des revendications précédentes, **caractérisée en ce que** ledit corps de lentille comporte des première et seconde parties évasées espacées l'une de l'autre le long dudit bord périphérique ;
un premier élément haptique attaché audit corps de lentille souple au niveau de ladite première partie évasée ; et
un second élément haptique attaché audit corps de lentille souple au niveau de ladite seconde partie évasée ;
dans laquelle ledit corps de lentille peut être plié suivant un axé entre lesdites première et seconde parties évasées.

9. Lentille intraoculaire selon la revendication 7 ou 8, **caractérisée en ce que** ledit corps de la lentille comporte une transition douce entre ledit bord périphérique et chaque partie évasée.

10. Procédé de formation d'une lentille intraoculaire, ledit procédé comprenant :
(1) l'utilisation d'un corps de lentille souple ayant une épaisseur globale maximale prédéterminée ;
(2) la formation d'une zone optique centrale dans ledit corps de lentille souple, la zone optique centrale présentant au moins une face convexe ayant un degré de courbure prédéterminé pour définir une épaisseur au centre entre la, au moins une, face convexe et une seconde face, opposée ; et
(3) la formation d'une zone périphérique dans le corps de lentille souple entourant la zone optique centrale, la zone périphérique comportant un bord d'une épaisseur inférieure à l'épaisseur au centre, **caractérisé en ce que** la zone périphérique comporte au moins une partie évasée ayant une épaisseur plus grande que l'épaisseur au bord périphérique restant.

11. Procédé selon la revendication 10, **caractérisé en ce qu'**un élément haptique est attaché à la, au moins une, partie évasée du corps de la lentille.

12. Procédé selon la revendication 10 ou 11, **caractérisé en ce qu'**au moins l'une des étapes de formation d'une zone optique centrale dans le corps de lentille souple et de formation d'une zone périphérique dans le corps de lentille souple comprend le fait de tourner et de couper le corps de la lentille sur un tour.

13. Procédé selon la revendication 10 ou 11, **caractérisé en ce qu'**au moins l'une des étapes consistant à utiliser un corps de lentille souple ayant une épaisseur globale maximale prédéterminée, à former une zone optique centrale dans le corps de lentille souple et à former une zone périphérique dans le corps de lentille souple comprend le moulage d'un corps de lentille mis en forme.

14. Procédé selon l'une quelconque des revendications 10 à 13, **caractérisé en ce que** le corps de lentille souple comprend une matière à mémoire de forme, et dans lequel ledit procédé comprend en outre le roulage de la lentille intraoculaire en une configuration compacte et la fixation temporaire de la lentille dans la configuration compacte pour l'implantation.
